(19) Europäisches Patentamt

European Patent Office

Office européen des brevets

(11) Veröffentlichungsnummer: **0 238 025 B1**

(12) **EUROPÄISCHE PATENTSCHRIFT**

(45) Veröffentlichungstag der Patentschrift: **30.12.92**

(51) Int. Cl.[5]: **A61K 6/08**

(21) Anmeldenummer: **87103809.7**

(22) Anmeldetag: **16.03.87**

(54) **Röntgenopake polymerisierbare Dentalmassen.**

(30) Priorität: **18.03.86 DE 3609038**

(43) Veröffentlichungstag der Anmeldung:
**23.09.87 Patentblatt 87/39**

(45) Bekanntmachung des Hinweises auf die
Patenterteilung:
**30.12.92 Patentblatt 92/53**

(84) Benannte Vertragsstaaten:
**BE CH DE FR GB LI NL**

(56) Entgegenhaltungen:
**EP-A- 0 189 540**
**DE-A- 3 421 155**

(73) Patentinhaber: **THERA Patent GmbH & Co. KG**
**Gesellschaft für industrielle Schutzrechte**
**Griesberg 2**
**W-8031 Seefeld 1(DE)**

(72) Erfinder: **Gasser, Oswald, Dr. Dipl.-Chem.**
**Hartstrasse 13**
**W-8031 Seefeld(DE)**
Erfinder: **Ellrich, Klaus, Dr. Dipl.-Chem.**
**Auingerstrasse 16**
**W-8031 Wörthsee(DE)**

(74) Vertreter: **Müller, Bernhard, Dr.**
**Graf-Toerring-Strasse 45**
**W-8031 Seefeld 2(DE)**

**Beschreibung**

Die Erfindung betrifft neue röntgenopake polymerisierbare Dentalmassen, insbesondere Zahnfüllmassen.

Ein Großteil der handelsüblichen Zahnfüllmassen besitzt nur eine schwache Röntgenabsorption. Hierdurch kann z. B. eine gelegte Zahnfüllung vom behandelnden Arzt mit den im zahnärztlichen Bereich verwendeten Röntgengeräten auf dem Röntgenbild nicht gesehen werden, bzw. nicht vom umgebenden Zahnmaterial unterschieden werden. Eine Überprüfung der gelegten Füllung insbesondere auch nach einer längeren Tragezeit ist somit nur noch oberflächlich möglich. Randspaltbildungen, Veränderungen in der umgebenden Zahnsubstanz (insbesondere unter der Füllung) sowie Materialverluste können vom Zahnarzt mit nicht röntgenopaken Dentalmassen nicht kontrolliert werden, was möglicherweise zu weiteren Schäden am Zahn des Patienten führen kann.

Außerdem können an schwer einsehbaren Stellen (z.B. Approximalbereich) auftretende Füllungsüberschüsse ohne Darstellung am Röntgenbildschirm oft schwer entdeckt werden.

Dieser Befund hat vor allem in jüngerer Zeit in zunehmendem Maße dazu geführt, daß eine Reihe von röntgenopaken polymerisierbaren Dentalmassen verkauft werden. Handelsübliche Präparate enthalten meistens Barium-, Strontium-, Lanthan- oder Zink-haltige Gläser, die beispielsweise aus den DE-OS 23 47 591, US-PS 3 808 170 und US-PS 3 975 203 bekannt sind, oder röntgenopake Zusatzstoffe zusammen mit anderen üblichen Füllstoffen wie etwa Quarz, bestimmten Lithiumaluminiumsilicaten, Kieselsäuren, Kieselgel oder Kieselsäuregranulaten. Röntgenopake Zusatzstoffe sind beispielsweise Bariumsulfat, Zirkondioxid oder Lanthanoxid. In den US-PS 3 971 754 und US-PS 3 801 344 werden keramische Füllstoffzusammensetzungen beschrieben, die u.a. auch Oxide von Lanthan, Hafnium und Seltenerdenmetallen enthalten. Röntgenopake Dentalmassen sollten für eine optimale Anwendung eine höhere Röntgensichtbarkeit als das menschliche Dentin haben. Üblicherweise gibt man die Röntgensichtbarkeit von Materialien in mm-Aluminium pro mm Material an. Das menschliche Dentin hat z. B. eine Röntgensichtbarkeit von ca. 1,5 mm Aluminium, d. h. röntgenopake dentale Werkstoffe sollten eine Röntgensichtbarkeit > 1,5 mm Aluminium haben.

Mit den oben erwähnten, bisher bekannten Zusatzstoffen und den keramischen Füllstoffzusammensetzungen erreicht man eine solche Röntgensichtbarkeit aber nur, wenn man gleichzeitig einen Verlust in der Transparenz der auspolymerisierten Massen mit in Kauf nimmt, was dazu führt, daß diese Massen in ihrem kosmetischen Erscheinungsbild dem umgebenden Zahnmaterial nicht mehr optimal angepaßt werden können. Die Transparenz der auspolymerisierten Massen hängt stark vom Verhältnis der Brechungsindizes der Füllkörper zur polymeren Matrix ab. Während sich die Brechungsindizes von Monomer und Polymer nicht stark unterscheiden - er liegt bei handelsüblichen polymerisierbaren Dentalmassen im Bereich 1,45 bis 1,6 - gibt es gerade bei den röntgenopaken Zusatzstoffen große Unterschiede, vor allem liegt er bei bisher bekannten röntgenopaken Zusatzstoffen über 1,6. Dentalmassen mit einer zu hohen Opazität haben aber nicht nur einen kosmetischen Nachteil, sondern führen bei Präparaten, die mit Licht ausgehärtet werden sollen, zudem noch zu einer ungenügenden Polymerisationstiefe, da das für die Aushärtung benötigte Licht nicht mehr genügend tief in die Masse eindringen kann. Dieses führt dann oft zu fehlerhaften Anwendungen, bei denen unter einer ausgehärteten Oberfläche noch nicht polymerisierte Substanz vorhanden ist, die dann eine weitere Schädigung der Zahnsubstanz nach sich ziehen kann. Außerdem wird die Qualität der ausgehärteten Masse durch darunter befindliche nicht ausgehärtete Bestandteile mangelhaft sein.

Aus diesem Grund enthalten die meisten handelsüblichen röntgenopaken Dentalmassen auch keine röntgenopaken Zusatzstoffe, sondern der gesamte oder zumindest ein sehr großer Anteil des Füllstoffgehaltes besteht aus röntgenopaken Gläsern, mit denen man eine ausreichende Transparenz bei befriedigender Röntgensichtbarkeit erreichen kann. Zahnfüllmassen mit diesen Gläsern haben allerdings nicht die physikalischen Eigenschaften wie sie mit anderen Füllstoffen erreicht werden können.Sie sind in der Regel hydrolytisch anfällig, das heißt, sie können im Laufe der Zeit an der Oberfläche herausgewaschen werden. Ihre Farbstabilität läßt zuweilen zu wünschen übrig und vor allem ihre physikalischen Eigenschaften fallen deutlich gegenüber mit anderen Füllstoffen wie z. B. Quarz gefüllten Composites ab. So sind mit Gläsern hergestellte Zahnfüllmassen aufgrund der geringeren Härte von Glas gegenüber Quarz weniger abrasionsstabil. Außerdem ist es nicht möglich, die Gläser so fein zu mahlen, daß man auch hochglanzpolierbare Zahnfüllmassen herstellen kann. Dieses gelingt bisher nur mit sogenannten Mikrofüller-Präparaten oder mit Zahnfüllmassen, die Granulate aus solchen sehr kleinen Primärteilchen enthalten. Eine hochglanzpolierbare Dentalmasse sollte nur Füllkörper mit einer Primärteilchengröße < 1 μm enthalten. Gläser, die man so fein mahlt, werden durch die hierzu nötigen Mahlprozesse opak, so daß wiederum die gleichen Nachteile wie bei den sonstigen röntgenopaken Zusatzstoffen auftreten.

Bezüglich des in den letzten beiden Absätzen geschilderten Sachverhalts wird auf EP-A-0 159 887, DE-A- 35 02 594 und R. Janda, "Die Quintessenz" 8/1988,S.1393 verwiesen.

Aufgabe der Erfindung ist daher die Bereitstellung von neuen röntgenopaken polymerisierbaren Zahnfüllmassen, die die Nachteile des Stands der Technik nicht aufweisen und bei denen insbesondere gleichzeitig eine ausgezeichnete Röntgensichtbarkeit und exzellente optische Eigenschaften verwirklicht sind.

Gegenstand der Erfindung sind röntgenopake polymerisierbare Dentalmassen, enthaltend ein oder mehrere ethylenisch ungesättigte polymerisierbare Monomere und/ oder Polymere sowie gegebenenfalls übliche Füllstoffe, Pigmente, Initiatoren, gegebenenfalls Aktivatoren und gegebenenfalls Thixotropiehilfsmitteln, wobei die Massen dadurch gekennzeichnet sind, daß sie zusätzlich ein schwer lösliches komplexes Schwermetallfluorid der allgemeinen Formel $M^{II}M^{IV}F_6$ oder $YF_3$ enthalten, wobei $M^{II}$ ein Calcium-, Strontium- oder Bariumion und $M^{IV}$ ein Titan-, Zirkon- oder Hafniumion bedeutet. wobei das schwerlösliche Schwermetallfluorid einen Brechungsindex von 1,45 - 1,60 aufweist.

Der Anteil an Schwermetallfluorid in diesen Massen beträgt vorzugsweise 5 - 30 Gew.-%, bezogen auf die Gesamtmasse. Vorzugsweise enthalten derartige Massen

a) bis zu 50 Gew.-% ethylenisch ungesättigtes polymerisierbares Monomer und/oder Polymer,

b) 30 - 70 Gew.-% übliche Füllstoffe, Pigmente und gegebenenfalls Thixotropiehilfsmittel und

c) 0,01 - 5 Gew.-% Polymerisationsinitiatoren,

wobei sich die Mengenangaben jeweils auf die Gesamtmasse beziehen.

Die erfindungsgemäßen röntgenopaken Zusatzstoffe können in einer Menge von insbesondere 10 - 30 Gew.-% und ganz besonders 10 - 20 Gew.-%, bezogen auf die Gesamtmasse, in der Zahnfüllmasse enthalten sein. Ein für die Praxis besonders geeigneter Anteil des Schwermetallfluorids liegt bei etwa 15 Gew.-%, bezogen auf die Gesamtmasse.

Die erfindungsgemäßen polymerisierbaren röntgenopaken Zahnfüllmassen haben den großen Vorteil, daß sie eine ausgezeichnete Röntgensichtbarkeit mit exzellenten optischen und kosmetischen Eigenschaften kombinieren. Hierbei soll einerseits die Zahnfüllmasse dem natürlichen Aussehen des Zahnes in Transparenz und Farbe angeglichen werden, andererseits ermöglicht die Unterscheidung von Zahnfüllung und Zahnschmelz im Röntgenbild die Beurteilung der Qualität der gelegeten Füllung.

Ein weiterer sehr großer Vorteil bei der erfindungsgemäßen Verwendung der Schwermetallfluoride ist, daß sie selbst bei relativ geringen Konzentrationen schon eine genügende Röntgensichtbarkeit der Dentalmassen erzielen, das heißt, daß man sie zu anderen Füllstoffen in relativ geringer Konzentration zudosieren kann, um die erforderliche Röntgenabsorption zu erzielen. Beim Zusatz solcher relativ geringen Mengen an Schwermetallfluoriden erhält man also Dentalmassen, deren physikalische Eigenschaften vom Hauptteil ihrer Füllkörper bestimmt sind und gleichzeitig röntgenopak sind. So kann man beispielsweise die Vorteile von Makrofüllern - großer Füllkörperanteil, dadurch geringer Schrumpf, kleiner thermischer Expansionskoeffizient, gute Abrasionsbeständigkeit - ausnützen und trotzdem durch Zusatz der erfindungsgemäßen Fluoride die Zahnfüllmassen röntgenopak machen.

Ein weiterer Vorteil bei Verwendung der erfindungsgemäßen Fluoride ergibt sich bei gleichzeitiger Verwendung von sogenannten Mikrofüllern. Da die Fluoride durch Fällungsreaktionen in fast jeder beliebigen Korngröße erhältlich sind, können hiermit zusammen mit den Mikrofüllern Pasten hergestellt werden, die röntgenopak und trotzdem hochglanzpolierbar sind. Ferner sind die erfindungsgemäßen Massen durch die Unlöslichkeit ihrer Bestandteile toxikologisch unbedenklich. Die erfindungsgemäß verwendeten Schwermetallfluoride besitzen so kleine Löslichkeitsprodukte, daß sie selbst beim Verschlucken durch den Patienten im Magen- und Darmmilieu nicht gelöst werden und somit nicht dem Organismus zugeführt werden können.

Zum besseren Einbau in die Polymermatrix kann es von Vorteil sein, nicht nur die üblichen Füllstoffe, sondern auch den röntgenopaken Zusatzstoff (Schwermetallfluorid) zu hydrophobieren. Übliche Hydrophobierungsmittel sind Silane, beispielsweise Trimethoxy-methacroyloxypropylsilan.

Ethylenisch ungesättigte Monomere bzw. Polymere, die für Dentalzwecke geeignet sind, umfassen beispielsweise monomere und polymere Acrylate und Methacrylate. Bei polymerisierbaren Dentalmassen verwendet man insbesondere oft die langkettigen Monomere der US-PS 3 066 112 auf der Basis von Bisphenol-A und Glycidyl-methacrylat oder dessen durch Addition von Isocyanaten entstandene Derivate. Besonders geeignet sind auch die Acrylsäure-bzw. Methacrylsäureester ein- oder mehrwertiger Alkohole, beispielsweise Methyl- und Ethylmethacrylat, Triethylenglykol-di-methacrylat und ähnliche. Besonders geeignet sind auch die in der DE-PS 2 816 823 genannten Diacryl- und Dimethacrylsäureester des Bis-hydroxymethyltricyclo-(5.2.1.0.$^{2,6}$)-decans. Verwendet werden können auch die Reaktionsprodukte aus Diisocyanaten und Hydroxyalkyl(meth)acrylaten wie sie beispielsweise in der DE-OS 2 312 559 beschrieben sind.

Selbstverständlich können auch Gemische aus geeigneten Monomeren bzw. hieraus hergestellt ungesättigte Polymere verwendet werden.

Dem Fachmann geläufige übliche Bestandteile der Zahnfüllmassen sind neben gesättigten oder ungesättigten Polymeren, Pigmente, Farbstoffe und anorganische Füllstoffe. Anorganische Füllstoffe können beispielsweise Quarz, gemahlene Gläser, Kieselgele sowie Kieselsäuren oder deren Granulate sein. Sie können in einer Konzentration von 0 - 90 Gew.- %, bezogen auf die polymerisierbare Masse, eingesetzt werden.

Geeignete Initiatorsysteme sind z.B. die für die Kalthärtung geeigneten Redoxsysteme,wie Peroxid/Amin oder Peroxid/Barbitursäurederivate u. ä. Bei Verwendung solcher Initiatorsysteme ist es zweckmäßig, eine Initiator-(z.B. Peroxid) und eine Katalysator-(z.B. Amin)Komponente zu unterscheiden. Der röntgenopake Füllstoff kann entweder in einer oder in beiden Komponenten enthalten sein.

Als Polymerisationsinitiatoren können aber auch Substanzen eingesetzt werden, die nach Bestrahlen durch UV oder sichtbares Licht die Polymerisation auslösen, beispielsweise Benzoinalkylether, Benzilmonoketale, Acylphosphinoxide oder aliphatische und aromatische 1,2-Diketo-verbindungen, z. B. Campherchinon, wobei die Lichtpolymerisation durch Zusatz von Aktivatoren, wie Aminen oder organischen Posphiten, in an sich bekannter Weise beschleunigt werden kann.

Nachstehend wird die Erfindung anhand von Beispielen näher erläutert.

Beispiel 1

Herstellung von Strontiumhexafluoro-zirkonat

Man löst 283,5 g (1 Mol) Kaliumhexafluorozirkonat in 10 l warmem Wasser und läßt die etwa 40°C warme Lösung durch eine Austauschersäule mit 2 l Kationenaustauscher in der H-Form (z.B. Relite CF$^R$)-laufen. Man versetzt das Filtrat mit 147 g (1 Mol) Strontiumcarbonat und rührt 16h bei RT. Am nächsten Tag saugt man ab, kocht den Niederschlag mit ca. 5 l Wasser 6h am Rückfluß und saugt dann heiß ab. Die gleiche Prozedur wird ein zweites Mal wiederholt. Den wiederum heiß abgesaugten Niederschlag trocknet man zunächst bei 120°C, schließlich bei 200°C.
Ausbeute:
181,5 g = 62 % d.Th.
Elementaranalyse:
Sr 30,05 % (ber. 29,92)
Zr 30,90 % (ber. 31,15)
Gegebenenfalls kann der vorstehend erwähnte Niederschlag nach dem Auskochen und Absaugen auch mit Aceton nachgewaschen und getrocknet werden.

Beispiel 2

Herstellung von Bariumhexafluoro-zirkonat

Man löst 141,8 g (0,5 Mol) Kaliumhexafluorozirkonat in 1 l heißem Wasser und tropft in der Hitze unter gutem Rühren zu: 122 g (0,5 Mol) Bariumchlorid-dihydrat, gelöst in 500 ml Wasser. Man rührt noch 30 Minuten bei 100°C nach, saugt dann ab und wäscht mit 1 l heißem Wasser nach. Zur Entfernung des Restkaliums wird insgesamt 5 x mit je 4 l Wasser jeweils 24h am Rückfluß gekocht. Nach dem letzten Absaugen wird getrocknet, zuletzt 2h im Vaccum bei 200°C.
Ausbeute:
85 g = 50 % d. Th.
Elementaranalyse:
Ba 40,35 % (ber. 40,09)
Zr 26,40 % (ber. 26,63)

Beispiel 3

Herstellung einer photopolymerisierbaren röntgenopaken Zahnfüllmasse

Aus 70 Gewichtsteilen Bisacryloxymethyltricyclo-(5.2.1.0.$^{2,6}$)-decan und 30 Gewichtsteilen 2,2-Bis-4-(3-methacryloxy-2-hydroxypropoxy)-phenylpropan (Bis-GMA), 7 Gewichtsteilen silanisierter pyrogener Kieselsäure, 0,3 Gewichtsteilen Campherchinon, 3 Gewichtsteilen N,N-Dimethylaminoethylmethacrylat und 110 Gewichtsteilen röntgenopakem Füllstoff wird eine Vormischung geknetet.

5,96 g dieser Vormischung werden mit insgesamt 12 g (Paste 1); bzw. 16 g (Paste 2), bzw. 14,8 g (Paste 4) silanisiertem und zahnähnlich pigmentierten Quarz (mittlere Korngröße ca. 6 $\mu$m) zu einer Zahnfüllmasse mit einheitlicher pastöser Konsistenz verknetet.

Als eine Vergleichspaste wird dieselbe Rezeptur, (Paste 3, 16 g Quarz) lediglich ohne röntgenopaken Füllstoff geknetet. Die Ergebnisse und physikalischen Meßwerte der zu erhaltenden Pasten sind in Tabelle 1 wiedergegeben. Paste 1 enthält Bariumzirkonfluorid, Paste 2 Strontiumzirkonfluorid und Paste 4 Yttriumfluorid als röntgenopaken Füllstoff. Paste 3 enthält keinen röntgenopaken Füllstoff (Vergleichspaste).

Tabelle 1

| Meßwert | Paste 1 | Paste 2 | Paste 3 (Vergleich) | Paste 4 |
|---|---|---|---|---|
| Schichtdicke | 8,5 mm | 8,3 mm | 8,5 mm | 8,3 mm |
| Druckfestigkeit | 316 MPa | 326 MPa | 320 MPa | 348 MPa |
| Biegefestigkeit | 102 MPa | 94 MPa | 92 MPa | 85 MPa |
| Röntgensichbarkeit | 3,0 mm Al | 2,3 mm Al | 0,5 mm Al | 1,8 mm Al |
| Opazität | 87,8 % | 87 % | 86 % | 87 % |

Die Schichtdicken werden in zylindrischen Körpern (Durchmesser 5 mm, Länge 8 mm) nach Bestrahlung mit einem handelsüblichen dentalen Bestrahlungsgerät (Elipar/Visio/Espe) nach 20 Sekunden gemessen. Hierzu wird das Polymerisat aus dem Zylinder genommen, die weichen oder gelartigen nicht durchpolymerisierten Bestandteile mit einem Kunststoffspatel entfernt und die erzielte Schichtdicke gemessen. Die Röntgensichtbarkeiten werden ermittelt, indem ein 1 mm hoher Prüfkörper aus polymerisiertem Material hergestellt wird und mit einer Aluminiumtreppe die Aluminiumhöhe ermittelt wird, die 1 mm Prüfmaterial entspricht. Die Opazitäten werden an einem CIELAB-Farbmessgerät anhand von Prüfkörpern mit 3,5 mm Höhe und 2 cm Durchmesser gemessen. Es zeigt sich, daß die erfindungsgemäßen röntgenopaken Massen zu Polymerisaten führen, die in ihren physikalischen Eigenschaften dem nicht röntgenopaken Material ebenbürtig sind und trotzdem eine für den dentalen Anwendungszweck ausreichende Röntgensichtbarkeit erzielen (Röntgensichtbarkeit von menschlichem Zahnschmelz 1,5 - 2,00 mm Aluminium).

Beispiel 4

Herstellung einer photopolymerisierbaren, röntgenopaken, hochglanzpolierbaren Zahnfüllmasse

45 Gew.-Teile Bis-(methacryloxymethyl)-tricyclo[5.2.1.0$^{2,6}$]-decan, 45 Gew.-Teile Bis-(acryloxymethyl)-tricyclo-[5.2.1.0$^{2,6}$]decan und 10 Gew.-Teile 2,2-Bis-4-(3-methacryloxy-2-hydroxypropoxy)-phenylpropan (Bis-GMA) werden unter vorsichtigem Erwärmen so lange gerührt, bis eine klare Lösung entsteht.

Zu der auf Raumtemperatur abgekühlten Lösung gibt man 0,15 Gew.-% Campherchinon und 1,5 Gew.-% N,N-Dimethylaminoethylmethacrylat und rührt so lange, bis eine klare Initiatorlösung vorliegt.

Aus 50 Gew.-Teilen dieser Initiatorlösung, 32 Gew.-Teilen Yttriumfluorid und 27 Gew.-Teilen silanisierter pyrogener Kieselsäure wird eine Vormischung geknetet.

35 Gew.-Teile dieser Vormischung werden mit 15 Gew.-Teilen silanisiertem Kieselsäuregranulat (EP-PS 0 040 232) zu einer Zahnfüllmasse mit einheitlicher, pastöser Konsistenz verknetet.

Füllt man die erhaltene röntgenopake Zahnfüllmasse in einen Zylinder (Durchmesser 5 mm, Länge 8 mm), belichtet 20 Sekunden mit einem handelsüblichen dentalen Bestrahlungsgerät (ELIPAR-VISIO/ESPE), nimmt anschliessend das Polymerisat aus dem Zylinder und entfernt die weichen und die gelartigen, nicht durchpolymerisierten Bestandteile mit einem Kunststoffspatel, so erhält man eine durchpolymerisierte Schicht von 6 mm. Die Röntgensichtbarkeit beträgt mehr als 2 mm Aluminium. Die Opazität beträgt 92 % und die Druckfestigkeit 385 MPa.

**Patentansprüche**

1. Röntgenopake polymerisierbare Dentalmasse, enthaltend ein oder mehrere ethylenisch ungesättigte polymerisierbare Monomere und/ oder Polymere sowie gegebenenfalls übliche Füllstoffe, Pigmente, Initiatoren, gegebenenfalls Aktivatoren und gegebenenfalls Thixotropiehilfsmittel, dadurch gekennzeichnet, daß sie zusätzlich ein schwerlösliches Schwermetallfluorid aus der Gruppe $YF_3$ und komplexe Schwermetallfluoride der allgemeinen Formel $M^{II}M^{IV}F_6$ enthält, wobei $M^{II}$ ein Calcium-, Strontium- oder Bariumion und $M^{IV}$ ein Titan-, Zirkon- oder Hafniumion bedeutet, wobei das schwerlösliche Schwermetallfluorid einen Brechungsindex von 1,45 - 1,60 aufweist.

**2.** Dentalmasse nach Anspruch 1, dadurch gekennzeichnet, daß der Anteil an Schwermetallfluorid 5 - 30 Gew.-%, bezogen auf die Gesamtmasse, beträgt.

**3.** Röntgenopake polymerisierbare Dentalmasse, nach Anspruch 2, dadurch gekennzeichnet, daß sie
a) bis zu 50 Gew.-% ethylenisch ungesättigtes polymerisierbares Monomer und/oder Polymer
b) 30 - 70 Gew.-% übliche Füllstoffe, Pigmente und gegebenenfalls Thixotropiehilfsmittel
c) 0,01 - 5 Gew.% Polymerisationsinitiatoren und gegebenenfalls Aktivatoren enthält, wobei sich die Mengenangaben jeweils auf die Gesamtmasse beziehen.

**4.** Dentalmasse nach einem der Ansprüche 1 bis 3, dadurch gekennzeichnet, daß das schwerlösliche komplexe Schwermetallfluorid $SrZrF_6$ oder $BaZrF_6$ ist .

**5.** Dentalmasse nach einem der Ansprüche 1 bis 3, dadurch gekennzeichnet, daß das schwerlösliche Schwermetallfluorid $YF_3$ ist.

**6.** Dentalmasse nach Anspruch 1 oder 3, dadurch gekennzeichnet, daß der Anteil an Schwermetallfluorid 10 - 20 Gew.-%, bezogen auf die Gesamtmasse, beträgt.

## Claims

**1.** An X-ray opaque, polymerisable dental composition containing one or more ethylenically unsaturated, polymerisable monomers and/or polymers, as well as optionally conventional fillers, pigments, initiators, optionally activators and optionally thixotropic auxiliary materials, characterized in that said composition further contains
a heavy-metal fluoride with very low solubility selected from the group consisting of $YF_3$ and complex heavy-metal fluorides having the general formula $M^{II}M^{IV}F_6$, where $M^{II}$ is a calcium, strontium or barium ion, and $M^{IV}$ is a titanium, zirconium or hafnium ion, wherein said heavy metal fluoride with very solubility has an index of refraction of 1.45-1.60.

**2.** A dental composition according to claim 1, characterised in that the amount of said heavy-metal fluoride is 5 - 30 % by weight of the total composition.

**3.** An X-ray opaque, polymerisable dental composition according to claim 2, characterised in that said composition contains:
(a) up to 50 % by weight ethylenically unsaturated polymerisable monomer and/or polymer,
(b) 30 - 70 % by weight conventional fillers, pigments, and optionally thixotropic auxiliary materials,
(c) 0.01 - 5 % by weight polymerisation initiators and optionally activators, with all said amounts being relative to the total composition.

**4.** A dental composition according to any of claims 1 to 3, characterised in that said complex heavy-metal fluoride with very low solubility is $SrZrF_6$ or $BaZrF_6$.

**5.** A dental composition according to any of claims 1 to 3, characterized in that said heavy metal fluoride with very low solubility is $YF_3$.

**6.** A dental composition according to claim 1 or 3, characterised in that the amount of said heavy metal fluoride is 10 - 20 % by weight of the total composition.

## Revendications

**1.** Pâte dentaire polymérisable opaque aux rayons X, contenant un ou plusieurs monomères et/ou polymères polymérisables éthyléniquement insaturés ainsi qu'éventuellement des matières de charge usuelles, pigments, amorceurs, le cas échéant des agents activants et éventuellement des adjuvants thixotropes, caractérisée en ce qu'elle contient en plus un fluorure difficilement soluble des métaux lourds du groupe $YF_3$ et des fluorures de métaux lourds complexes de la formule générale $M^{II}M^{IV}F_6$, $M^{II}$ signifiant un ion de calcium, de strontium ou de baryum et $M^{IV}$ un ion de titane, de zircone ou d'hafnium, le fluorure difficilement soluble des métaux lourds présentant un indice de réfraction de 1,45 - 1,60.

2. Pâte dentaire selon la revendication 1, caractérisée en ce que la proportion en fluorure de métaux lourds est de 5 - 30 % en poids rapportés à la masse totale.

3. Pâte dentaire polymérisable opaque aux rayons X, selon la revendication 2, caractérisée en ce qu'elle contient
   a) jusqu'à 50 % en poids de monomère et/ou polymère polymérisables insaturés éthyléniquement
   b) 30 - 70 % en poids de matière de charge usuelle, pigments et éventuellement adjuvants thixotropes
   c) 0,01 - 5 % en poids d'amorceurs de polymérisation et éventuellement d'agents activants, les indications quantitatives se rapportant chaque fois à la masse totale.

4. Pâte dentaire selon l'une des revendications 1 à 3, caractérisée en ce que le fluorure difficilement soluble des métaux lourds complexes est $SrZrF_6$ ou $BaZrF_6$.

5. Pâte dentaire selon l'une des revendications 1 à 3, caractérisée en ce que le fluorure difficilement soluble des métaux lourds est $YF_3$.

6. Pâte dentaire selon la revendication 1 ou 3, caractérisée en ce que la quantité en fluorure de métaux lourds est de 10-20 % en poids rapportés à la masse globale.